Europäisches Patentamt

(19) European Patent Office

Office européen des brevets

(11) **EP 0 761 190 B1**

(12) **EUROPEAN PATENT SPECIFICATION**

(45) Date of publication and mention
of the grant of the patent:
**24.11.1999 Bulletin 1999/47**

(51) Int. Cl.$^6$: **A61F 13/15**

(21) Application number: **95120496.5**

(22) Date of filing: **22.12.1995**

(54) **Sanitary articles with multi aperture size film topsheets having a selected distribution of the apertures**

Eine, mit in Grösse unterschiedlicher und definierter Verteilung der Perforationen, versehene Decklage für Hygieneartikel

Article d'hygiène avec une couche supérieure à perforations de dimensions multiples et une distribution sélectionnée de perforations

(84) Designated Contracting States:
**AT BE CH DE ES FR GB IT LI LU NL SE**

(30) Priority: **06.09.1995 EP 95113943**

(43) Date of publication of application:
**12.03.1997 Bulletin 1997/11**

(73) Proprietor:
**THE PROCTER & GAMBLE COMPANY
Cincinnati, Ohio 45202 (US)**

(72) Inventor:
**Hirsch, Uwe Thomas Michael Horst
D-64347 Griesheim (DE)**

(74) Representative:
**Hirsch, Uwe Thomas et al
Procter & Gamble European Service GmbH,
Sulzbacher Strasse 40-50
65824 Schwalbach am Taunus (DE)**

(56) References cited:
EP-A- 0 018 020      EP-A- 0 165 807
EP-A- 0 205 286      EP-A- 0 523 719
EP-A- 0 565 717      WO-A-93/12749

Printed by Xerox (UK) Business Services
2.16.7/3.6

## Description

### Field of the invention

The present invention relates to absorbent articles particularly sanitary napkins preferably having film topsheets, see EP-A-0523 719. In particular a topsheet receiving the liquids to be absorbed comprises apertures of at least 4 different sizes which are distributed according to their respective size in longitudinal and transverse directions. This range of aperture sizes provides exceptionally good liquid intake performance for the range of liquid viscosities commonly found for liquids which are absorbed in sanitary napkins. The distribution of the apertures leaves a certain longitudinal side edge and preferably transverse end edge of the topsheet free of the larger apertures to improve comfort of the product during use.

### Background of the invention

Sanitary articles such as sanitary napkins, baby diapers, absorbent inserts, and absorbent adult incontinence articles are well-known in the art. Typically all these articles comprise a wearer facing surface and a garment facing surface. The wearer facing surface receives from the wearer of such articles liquids, bodily discharges such as urins, vaginal discharges or menses, to be absorbed. In order for the article to store the liquid the wearer facing surface has to be liquid permeable while maintaining integrity of the outer wearer facing surface of the absorbent article. This wearer facing surface is provided by a topsheet.

Well-known topsheets in the art of absorbent articles are non-woven fabrics, woven fabrics or films. Films have to be rendered permeable by aperturing. They are often made of polymeric material and typically comprise apertures which have been engineered to provide certain characteristics. These apertures can vary in shape and size but have commonly been provided in a single preferred size and shape. The walls of the apertures define the amount of extension-if any- beyond the plane of the film thickness and the direction of such extensions. The film apertures also can be provided in the shape of a funnel. Films are generally preferred over fabrics since they can provide a cleaner surface even after liquid having passed through since they do not retain liquids. A typical topsheet made of polyethylene film has been successfully used in sanitary articles and adult incontinence products as well as inserts and baby diapers by Procter & Gamble under the tradenames Always and Attends.

Fabrics or non-woven fabrics are made of fibers which by their nature provide non-linear apertures in the liquid transport direction. Also the largest aperture size in fabric topsheets is limited by the requirement to maintain material strength. However, these materials can also be treated in a similar fashion as film materials in particular non-woven fabrics which are made of thermo-plastic fibers. Aperturing of fabrics or non-wovens is hence also considered by the present invention.

One problem remaining with all these topsheets is the rate of liquid capable of passing through such a topsheet under usual usage conditions due to the total amount of open area of all apertures and individual aperture size and shape in particular. Exceptionally large apertures increase the liquid passage rate but pose the potential problem of material stability for the topsheet at least during the manufacturing of absorbent articles, which is highly undesirable. Also large apertures possibly promote a backflow of absorbed liquid, so called rewet, which is undesirable. Small individual apertures on the other hand cannot provide the liquid passage characteristics required to let liquids of high surface tension, high viscosity or solid content such as coagulated blood clots pass through; this can be a problem in an absolute sense for very small apertures or cause too low a rate of liquid flow.

It also has been found that the total amount of open area for a given aperture size and shape is approximately linearly related to the rate of liquid passage. Again, masking of the liquid which has passed through but also material strength and other appearance considerations are limiting the extend as to which the total open area in a topsheet can be selected.

It is also known that the viscosity of liquids disposed on sanitary napkins can very drastically from exceptionally low viscosities e.g. of urine from a person having a light incontinence or stress incontinence syndrome. Low viscosities also can be present for vaginal discharges or menses which comprise small amounts of surfactant. Some bodily discharges comprise natural surfactants already but also residue from washing soaps, which many wearers of such articles use regularly, can be found.

At the other end of the spectrum viscosities of menses changes drastically over the period of a woman and have been reported in EP-A-0 205 286 to range from 5 to 50 mPas. The viscosities can change over time on the same person and can vary between persons compared at a similar stage of the monthly period.

Another problem which now has been recognized is that the presence of large hole apertures in the longitudinal side margins or the transverse end regions of the wearer facing surface of a topsheet can cause undesirable chaffing or irritation. This is believed to be possibly due to the amount of material forming the rim of the aperture being more for larger apertures than for smaller apertures. It is well-known that even tiny points of irritation from such an article will cause significant discomfort. In fact wearers claiming to experience discomfort typically will disregard any absorbency benefits obtained by the multi aperture topsheet due to unacceptable comfort performance of such articles.

It is therefore an objective of the present invention to provide a topsheet for sanitary articles which provide the benefits previously recognized by inclusion of an aperture distribution in the topsheet while eliminating the problems associated with having large apertures in a region where relative movement between the topsheet and the skin of a wearer would potentially cause discomfort.

It is another an objective of the present invention to provide a topsheet for sanitary articles which can even better accommodate the viscosity changes of absorbent liquids without the need for different articles for different times. It has been found that this can be achieved by providing topsheets for absorbent disposable articles which have apertures of different open areas in an aperture distribution relating to the individual aperture open area.

It is hence an objective of the present invention to provide absorbent articles which have a topsheet with a distribution of aperture sizes and locations.

**Description of the invention**

The present invention provides an absorbent article according to claim 1 having the benefit of an aperture topsheet for liquids of drastically different viscosities without discomfort to the wearer. In particular, the absorbent article comprises a topsheet having a wearer facing surface and a garment facing surface. The topsheet can comprise multiple layers which are preferably joined to each other. An absorbent article generally further comprises a backsheet and an absorbent structure, alternatively called absorbent core, placed between the topsheet and the backsheet.

Absorbent structure

The absorbent structure can include the following components: (a) optionally a primary fluid distribution layer preferably together with a secondary optional fluid distribution layer; (b) a fluid storage layer; (c) optionally a fibrous ("dusting") layer underlying the storage layer; and (d) other optional components.

a Primary/Secondary Fluid Distribution Layer

One optional component of the absorbent structure according to the present invention is a primary fluid distribution layer and a secondary fluid distribution layer. The primary distribution layer typically underlies the topsheet and is in fluid communication therewith. The topsheet transfers the acquired fluid to this primary distribution layer for ultimate distribution to the storage layer. This transfer of fluid through the primary distribution layer occurs not only in the thickness, but also along the length and width directions of the absorbent product. The also optional but preferred secondary distribution layer typically underlies the primary distribution layer and is in fluid communication therewith. The purpose of this secondary distribution layer is to readily acquire fluid from the primary distribution layer and transfer it rapidly to the underlying storage layer. This helps the fluid capacity of the underlying storage layer to be fully utilised. The fluid distribution layers can be comprised of any material typical for such distribution layers. In particular fibrous layers maintain the capillaries between fibers even when wet are useful as distribution layers.

b Fluid Storage Layer

Positioned in fluid communication with, and typically underlying the primary or secondary distribution layers, is a fluid storage layer. The fluid storage layer can comprise any usual absorbent material or combinations thereof. It preferably comprises absorbent gelling materials usually referred to as "hydrogel" "superabsorbant", hydrocolloid" materials in combination with suitable carriers.

The absorbent gelling materials are capable of absorbing large quantities of aqueous body fluids, and are further capable of retaining such absorbed fluids under moderate pressures. The absorbent gelling materials can be dispersed homogeneously or non-homogeneously in a suitable carrier. The suitable carriers, provided they are absorbent as such, can also be used alone.

Suitable absorbent gelling materials for use herein will most often comprise a substantially water-insoluble, slightly cross-linked, partially neutralised, polymeric gelling material. This material forms a hydrogel upon contact with water Such polymer materials can be prepared form polymerizable, unsaturated, acid-containing monomers which are well known in the art.

Suitable carriers include materials which are conventionally utilised in absorbent structures such as natural, modified or synthetic fibers, particularly modified or non-modified cellulose fibers, in the form of fluff and/or tissues. Suitable carriers can be used together with the absorbent gelling material, however, they can also be used alone or in combinations. Most preferred are tissue or tissue laminates in the context of sanitary napkins/panty liners.

An embodiment of the absorbent structure made according to the present invention comprises a double layer tissue laminate formed by folding the tissue onto itself. These layers can be joined to each other for example by adhesive or by mechanical interlocking or by hydrogen bridge bands. Absorbent gelling material or other optional material can be comprised between the layers.

Modified cellulose fibers such as the stiffened cellulose fibers can also be used. Synthetic fibers can also be used and include those made of cellulose acetate, polyvinyl fluoride, polyvinylidene chloride, acrylics (such as Orlon), polyvinyl acetate, non-soluble polyvinyl alcohol, polyethylene, polypropylene, polyamides (such as nylon), polyesters, bicomponent fibers, tricomponent fibers, mixtures thereof and the like. Preferably, the fiber surfaces are hydrophilic or are treated to be hydrophilic. The storage layer can also include filler materials, such as Perlite, diatomaceous earth, Vermiculite, etc., to improve liquid retention.

If the absorbent gelling material is dispersed non-homogeneously in a carrier, the storage layer can nevertheless be locally homogenous, i.e. have a distribution gradient in one or several directions within the dimensions of the storage layer. Non-homogeneous distribution can also refer to laminates of carriers enclosing absorbent gelling materials partially or fully.

c <u>Optional Fibrous ("Dusting") Layer</u>

An optional component for inclusion in the absorbent structure according to the present invention is a fibrous layer adjacent to, and typically underlying the storage layer. This underlying fibrous layer is typically referred to as a "dusting", layer since it provides a substrate on which to deposit absorbent gelling material in the storage layer during manufacture of the absorbent structure. Indeed, in those instances where the absorbent gelling material is in the form of macro structures such as fibers, sheets or strips, this fibrous "dusting" layer need not be included. However, this "dusting" layer provides some additional fluid-handling capabilities such as rapid wicking of fluid along the length of the pad.

d <u>Other Optional Components of the absorbent structure</u>

The absorbent structure according to the present invention can include other optional components normally present in absorbent webs. For example, a reinforcing scrim can be positioned within the respective layers, or between the respective layers, of the absorbent structure. Such reinforcing scrims should be of such configuration as to not form interfacial barriers to fluid transfer. Given the structural integrity that usually occurs as a result of thermal bonding, reinforcing scrims are usually not required for thermally bonded absorbent structures.

Another component which can be included in the absorbent structure according to the invention and preferably is provided close to or as part off the primary or secondary fluid distribution layer are odor control agents. Active carbon coated with or in addition to other odor control agents, in particular suitable zeolite or clay materials, are optionally incorporated in the absorbent structure. These components can be incorporated in any desired form but often are included as discrete particles.

<u>Backsheet</u>

The backsheet primarily prevents the exudates absorbed and contained in the absorbent structure from wetting articles that contact the absorbent product such as underpants, pants, pyjamas and undergarments. The backsheet is preferably impervious to liquids (e.g. menses and/or urine) and is preferably manufactured from a thin plastic film, although other flexible liquid impervious materials can also be used. As used herein, the term "flexible" refers to materials that are compliant and will readily conform to the general shape and contours of the human body. The backsheet also can have elastic characteristics allowing it to stretch in one or two directions.

The backsheet typically extends across the whole of the absorbent structure and can extend into and form part of or all of the preferred sideflaps, side wrapping elements or wings.

The backsheet can comprise a woven or nonwoven material, polymeric films such as thermoplastic films of polyethylene or polypropylene, or composite materials such as a film-coated nonwoven material. Preferably, the backsheet is a polyethylene film having a thickness of from about 0.012 mm (0.5 mil) to about 0.051 mm (2.0 mils).

Exemplary polyethylene films are manufactured by Clopay Corporation of Cincinnati, Ohio, under the designation P18-0401 and by Ethyl Corporation, Visqueen Division, of Terre Haute, Indiana, under the designation XP-39385. The backsheet is preferably embossed and/or matte finished to provide a more clothlike appearance. Further,. the backsheet can permit vapours to escape from the absorbent structure, i.e. be breathable, while still preventing exudates from passing through the backsheet. Also breathable backsheets comprising several layers, e.g. film plus non-woven structures, can be used.

The topsheet

For multiple layer topsheets the term "joined", as used herein, encompasses configurations in which the first layer is directly secured to the second layer by affixing the first layer directly to the second layer; configurations in which the first layer is indirectly secured to the second layer by affixing the first layer to intermediate layer(s) which in turn are affixed to the second layer:

The layers of the topsheet can preferably be joined together by adhesives, stitching, heat and/or pressure bonds, dynamic mechanical bonds, ultrasonic bonds, intermingling or entanglement of structural elements comprising the layers of the topsheet, such as by extruding one layer onto another, or by any other means known in the art. It is, however, important that the joining does not create any harsh or irritating spots on the topsheet.

The topsheet as a whole and hence each layer individually needs to be compliant, soft feeling, and non-irritating to the wearer's skin. It also can have elastic characteristics allowing it to be stretched in one or two directions. The topsheet typically extends across the whole absorbent structure and can extend into and form part of or all of the preferred side flaps, side wrapping elements or wings.

According to the present invention preferably at least one layer of the topsheet is provided by a film material having apertures which are referred herein as "extra large apertures", "large apertures", "medium apertures" and "small apertures". These apertures are provided to facilitate liquid transport for liquids of various viscosities from the wearer facing surface towards the absorbent structure. The apertures may also be present in a fabric or non-woven layer of a topsheet. In the following reference to a film topsheet is by way of example and application of the present invention to a fabric or non-woven fibrous topsheet is considered within the scope of the present invention.

For all measurements regarding the apertures in the film passage layer the plane of the smallest cross sectional areas of the aperture should be used, unless otherwise mentioned. The term "aperture size" as used herein refers to the open area which an aperture provides in the film.

The "extra large apertures" have an individual open area of from more than $1.4 \text{ mm}^2$, to $3 \text{ mm}^2$. The total open area of the "extra large apertures" excluding all other liquid transport apertures should be in the range from 1 % to 25 %, preferably from 10 % to 20 % of the surface area of the topsheet.

The "large apertures" have an individual open area of from more than $0.5 \text{ mm}^2$, to $1.4 \text{ mm}^2$. The total open area of the "large apertures" excluding all other liquid transport apertures should be in the range from 1 % to 30 %, preferably from 5 % to 15 % of the surface area of the topsheet.

The "medium apertures" have an individual open area of from more than $0.1 \text{ mm}^2$, to $0.5 \text{ mm}^2$. The total open area of the "medium apertures" excluding all other liquid transport apertures should be in the range from 1 % to 35 %, preferably from 10 % to 25 % of the surface area of the topsheet.

The "small apertures" have an individual open area of from more than $0 \text{ mm}^2$, to $0.1 \text{ mm}^2$. The total open area of the "small apertures" excluding all other liquid transport apertures should be in the range from 0.1 % to 5 %, preferably from 0.5 % to 3 % of the surface area of the topsheet.

The apertures are preferably substantially circular or polygonal. Their shape is limited by having a ratio of the largest to the smallest inner diagonal length in the range between 1 and 6, preferably 1 and 3. The total open area of all liquid transport apertures is in the range of 3.1 % to 95 %, preferably 10 % to 50 %, most preferably 15 % to 40 % of the total area of the topsheet.

Topsheets according to the present invention have a non-homogeneous distribution of the liquid passage ways or apertures across the wearer facing surface of the topsheet. It recently has been found that a topsheet comprising large and extra large apertures have a higher tendency of being considered to a cause discomfort to some wearers of sanitary napkins comprising such topsheets.

While not wishing to be bound by theory it is considered that the following is an explanation of the reasons for the benefits obtained with the present invention. When creating apertures, e.g. by a thermic process the material initially filling the aperture is not eliminated and ultimately it is collected in the rim around the aperture. This is considered equally true for fibrous non-woven topsheet layers or film topsheet layers. This rim becomes more apparent to a wearer the larger the apertures are since the open area of an aperture increases faster than the length of the aperture rim with increasing aperture diameters. The additional material deposited in the rim of the aperture hence builds up more in larger apertures than in smaller apertures. However, with increasing rim material the rim also becomes harsher and harder. Therefore the rim of larger apertures is more prone to cause irritation to the wearer of an article comprising such an apertured topsheet than for small apertures.

The absorbent article and all main components have a longitudinal axis which is parallel to a plane which would bisect a wearer of such an article into right and left side. Also the absorbent article has a transverse axis which is perpendicular to the longitudinal axis and within the same plane as the article. The term "longitudinal center" as used herein refers to the center of the article and extends parallel to the longitudinal axis. The term "transverse center" as used herein refers to the center of the article and extends parallel to the transverse axis.

It hence has been found that a preferable distribution of the apertures should leave the longitudinal margins of the topsheet free of extra large and large apertures. Hence the topsheet according to the present invention requires that the extra-large apertures mentioned above are only distributed in the longitudinal center 90 % of the largest transverse width of the absorbent structure. Preferably, these extra large apertures are distributed in the longitudinal center 80 %, most preferably 70 % of the largest transverse width of the absorbent structure.

In a preferred embodiment also the large apertures are eliminated from the longitudinal side margins of the topsheet such that the large apertures are only distributed in the longitudinal center 90 %, preferably 80 %, most preferably 70 % of the largest transverse width of the absorbent structure.

Optionally for ease of manufacturing of the apertured topsheet it is possible to only aperture the topsheet in the region of the longitudinal center to satisfy the specific requirement stated above for extra large and large apertures.

In a similar consideration it is also preferable that an embodiment of the present invention has the extra large apertures only distributed in the transverse center 80 %, preferably 65 %, most preferably 50 % of the largest longitudinal length of the wearer facing surface of the topsheet. Similarly large apertures are preferably only distributed in the transverse center 80 %, preferably 65 %, most preferably 50 % of the largest longitudinal length of the wearer facing surface of the topsheet.

As with the distribution in transverse direction for ease of manufacturing of a topsheet according to the present invention it is possible to provide the liquid transport apertures only in the transverse center of the topsheet in order to satisfy the above requirements for embodiments in the context of extra large and large apertures.

An additional benefit obtainable from the basic or preferred embodiments according to the present invention is the concentration of liquid apertures towards the center of the absorbent article such that more open area is placed where liquid acquisition is most probable. This then provides a benefit in absorbency of the respective absorbent article.

The liquid transport apertures can be simple holes but preferably are formed in the film such that the walls of the apertures extend beyond the plane of the surface of the basic film, i.e. the film surface, before the film is apertured. The direction of these extending walls in the absorbent article is towards the garment facing surface of the article. The amount of extension of the walls of the apertures should be at least 0.3 mm beyond the film surface from which the walls of the apertures depend. Preferably the walls of the apertures form funnels or Ventury channels as is well-known in the art.

To ensure material stability the smallest distance between neighbouring extra large and/or large apertures regardless of their particular shape and size is preferably at least 0.5 mm, preferably 1.5 mm. This distance is measured on the surface of the film on the side closest to the user facing surface of the absorbent article.

The film material is preferably rendered hydrophilic to such a degree that the contact angle is less than 90° with distilled water upon first contact with the water. For films this can be achieved by surfactant treatment. For surfactant treated polymeric films it has been found that it is beneficial to use films where the surfactant is permanently fixed on the film surface. These are so called film materials with resin integrated surfactant. For these films even repeated wetting by distilled water would provide approximately the same contact angle as the first contact with distilled water.

In another preferred execution the wearer facing surface of the topsheet is treated with an agent such that liquids are directed towards the apertures. Such agents can be silicone or teflon which provide the treated surface with a self-cleaning effect. This treatment can be in addition to the above-mentioned surfactant treatment.

Films such as those disclosed in EP-A-0 205 286, EP-A-0 165 208, EP-A-0 18 020, EP-A-0 59 506 or US-A-3,929,135 are explicitly referred to as suitable for the topsheet provided the requirements for the aperture distribution is met. Other suitable formed films, also provided the requirements for the aperture distribution are met, are described in EP-A-203 820, U.S. Patent 4,324,246, U.S. Patent 4,342,314, U.S. Patent 4,463,045 and U.S. Patent 5,006,394. Particularly preferred microaperturing of formed film is disclosed in U.S. patent 4,609,518 and U.S. patent 4,629,643. These microapertures can also be included in the topsheet provided their surface is less than 0.15 mm$^2$ and hence they essentially provide breathability. Ways of making such films are well-known in the art and have also been disclosed in the above prior art references. Also films which are, prior to aperturing, water vapour permeable but liquid impermeable can be used in the context of the present invention.

If the topsheet comprises multiple layers the second and following layers can be of the same kind as the above described topsheet according to the present invention with an aperture distribution. They can also be provided by other film layers or by woven or non-woven layers.

It is recommendable that any additional layer presents no barrier for the liquid. This can be achieved by providing no hydrophilicity gradient or an absorbency driving hydrophilicity gradient such that a directing force for the liquid towards the absorbent structure is created. It is also considered desirable that all layers have about the same total open area.

While not wishing to be limited by theory it is believed that a constant Reynolds number could ensure optimum liquid passage rates. The optimum Reynolds number depends of course on the absorption speed of the absorbent core. Once this Reynolds number is established for a particular liquid viscosity, absorbent core and one apertures size, the theoretically ideal distribution of aperture sizes can be identified by the equation.

$$\text{Reynolds number} = \frac{\text{density} \cdot \text{flow speed} \cdot \text{hydraulic diameter}}{\text{viscosity}}$$

wherein

- density is the density of the liquid to be absorbed in units of mass over volume,
- flow speed is the velocity of the liquid to be absorbed in units of distance over time,
- hydraulic diameter can be calculated from the size of the aperture as the square route of 4 times the aperture size over Pi.
- viscosity is the dynamic viscosity of the liquid to be absorbed in units of pressure times time.

With density being approximately constant (about that of water) and flow speed being constant by desire the hydraulic diameter distribution becomes approximately linearly proportional to the viscosity and the aperture size distribution becomes approximately proportional to the square of the viscosity:

$$\text{aperture size} \sim \text{viscosity}^2$$

Following this theory it is easy to select for a viscosity profile, which is expected over the usage conditions for a certain topsheet the aperture size distribution which is theoretically ideal. From this theoretically ideal distribution a realistic approximation with at least 4 different aperture sizes can be selected to provide a topsheet according to the present invention.

Preferred topsheet according to the present invention can have a larger number than 4 apertures, preferably the "theoretically ideal distribution of apertures" is resembled by a distribution of 5, 6, 7, 8, 9 or even 10 different aperture sizes. The majority of benefits from an aperture size distribution are however already realized by 5, 6, 7 or 8 different aperture sizes. The distribution must still satisfy the distribution given for 4 apertures, preferably with a skew towards the center of the distribution.

## Claims

1.  Absorbent article having a longitudinal axis and a transverse axis, said article comprising a topsheet, a backsheet, and an absorbent structure placed between said topsheet and said backsheet, said topsheet having a wearer facing surface and a garment facing surface and said topsheet comprising a film passage layer having small, medium, large and extra-large apertures for liquid transport,

    - said small apertures having an individual area in the range from more than 0 mm$^2$ to 0.1 mm$^2$,
    - said medium apertures having an individual area in the range from more than 0.1 mm$^2$ to 0.5 mm$^2$,
    - said large apertures having an individual area in the range from more than 0.5 mm$^2$ to 1.4 mm$^2$,
    - said extra-large apertures having an individual area in the range from more than 1.4 mm$^2$ to 3 mm$^2$,
    - said small apertures having a total open area in the range from 0.1 % to 5 % of the total area of said film passage layer,
    - said medium apertures having a total open area in the range from 1 % to 35 % of the total area of said film passage layer,
    - said large apertures having a total open area in the range from 1 % to 30 % of the total area of said film passage layer,
    - said extra-large apertures having a total open area in the range from 1 % to 25 % of the total area of said film passage layer,
    - said liquid transport apertures having a largest inner diagonal length and a smallest inner diagonal length, the ratio of said largest to said smallest inner diagonal length being in the range from 1 to 6,
    - said apertures following a distribution such that said extra large apertures are only distributed in the longitudinal center 90 % of the largest transverse width of said absorbent structure.

2.  Absorbent article according to claim 1 wherein at least some of said liquid transport apertures have inner walls which depend at least 0.3 mm from the surface of said film passage layer, said inner walls depend in a direction towards said absorbent structure of said article.

3.  Absorbent article according to any of the preceding claims wherein the total open area of all said liquid transport apertures in said film passage layer is in the range form 15 % to 40 % of the total area of said film passage layer.

4.  Absorbent article according to any of the preceding claims wherein said topsheet comprises more than one passage layer.

5. Absorbent article according to any of the preceding claims wherein said extra large apertures are only distributed in the longitudinal center 80 %, preferably 70 %, of said largest transverse width of said absorbent structure.

6. Absorbent article according to any of the preceding claims wherein said large apertures are only distributed in the longitudinal center 90 %, preferably 80 %, most preferably 70 %, of said largest transverse width of said absorbent structure.

7. Absorbent article according to any of the preceding claims wherein said liquid transport apertures are only distributed in the longitudinal center of said topsheet.

8. Absorbent article according to any of the preceding claims wherein all said extra large apertures are only distributed in the transverse center 80 %, preferably 65 %, most preferably 50 %, of the largest longitudinal length of said wearer facing surface of said topsheet.

9. Absorbent article according to any of the preceding claims wherein said large apertures are only distributed in the transverse center 80 %, preferably 65 %, most preferably 50 %, of the largest longitudinal length of said wearer facing surface of said topsheet.

10. Absorbent article according to any of the preceding claims wherein said liquid transport apertures are only distributed in the transverse center of said topsheet.

**Patentansprüche**

1. Absorbierender Artikel mit einer Längsachse und einer Querachse, wobei der genannte Artikel ein Deckblatt, ein Rückenblatt und eine zwischen dem genannten Deckblatt und dem genannten Rückenblatt angeordnete Struktur umfaßt, das genannte Deckblatt eine zum Träger gerichtete Oberfläche und eine zur Kleidung gerichtete Oberfläche aufweist und das genannte Deckblatt eine Foliendurchgangsschichte umfaßt, die kleine, mittlere, große und extra-große Öffnungen für den Flüssigkeitstransport aufweist, wobei

   - die genannten kleinen Öffnungen eine Einzelfläche im Bereich von mehr als 0 mm$^2$ bis 0,1 mm$^2$ aufweisen,
   - die genannten mittleren Öffnungen eine Einzelfläche im Bereich von mehr als 0,1 mm$^2$ bis 0,5 mm$^2$ aufweisen,
   - die genannten großen Öffnungen eine Einzelfläche im Bereich von mehr als 0,5 mm$^2$ bis 1,4 mm$^2$ aufweisen,
   - die genannten extra-großen Öffnungen eine Einzelfläche im Bereich von mehr als 1,4 mm$^2$ bis 3 mm$^2$ aufweisen,
   - die genannten kleinen Öffnungen eine gesamte offene Fläche im Bereich von 0,1 % bis 5 % der gesamten Fläche der genannten Foliendurchgangsschichte aufweisen,
   - die genannten mittleren Öffnungen eine gesamte offene Fläche im Bereich von 1 % bis 35 % der gesamten Fläche der genannten Foliendurchgangsschichte aufweisen,
   - die genannten großen Öffnungen eine gesamte offene Fläche im Bereich von 1 % bis 30 % der gesamten Fläche der genannten Foliendurchgangsschichte aufweisen,
   - die genannten extra-großen Öffnungen eine gesamte offene Fläche im Bereich von 1 % bis 25 % der gesamten Fläche der genannten Foliendurchgangsschichte aufweisen,
   - die genannten Flüssigkeitstransportöffnungen eine größte innere diagonale Länge und eine kleinste innere diagonale Länge aufweisen, wobei das Verhältnis der genannten größten zu der genannten kleinsten inneren diagonalen Länge im Bereich von 1 bis 6 liegt,
   - wobei die genannten Öffnungen einer solchen Verteilung entsprechen, daß die genannten extra-großen Öffnungen nur in der Längsmitte auf 90 % der größten Querbreite der genannten absorbierenden Struktur verteilt sind.

2. Absorbierender Artikel nach Anspruch 1, in welchem mindestens einige der genannten Flüssigkeitstransportöffnungen innere Wände haben, die von der Oberfläche der genannten Foliendurchgangsschichte mindestens 0,3 mm herabhängen, wobei die genannten inneren Wände in einer Richtung zu der genannten absorbierenden Struktur des genannten Artikels herabhängen.

3. Absorbierender Artikel nach einem der vorhergehenden Ansprüche, in welchem die gesamte offen Fläche aller genannten Flüssigkeitstransportöffnungen in der genannten Foliendurchgangsschichte im Bereich von 15 % bis 40 % der gesamten Fläche der genannten Foliendurchgangsschichte liegt.

**4.** Absorbierender Artikel nach einem der vorhergehenden Ansprüche, in welchem das genannte Deckblatt mehr als eine Durchgangsschichte umfaßt.

**5.** Absorbierender Artikel nach einem der vorhergehenden Ansprüche, in welchem die genannten extra-großen Öffnungen nur in der Längsmitte auf 80 %, vorzugsweise 70 %, der genannten größten Querbreite der genannten absorbierenden Struktur verteilt sind.

**6.** Absorbierender Artikel nach einem der vorhergehenden Ansprüche, in welchem die genannten großen Öffnungen nur in der Längsmitte auf 90 %, vorzugsweise 80 %, am bevorzugtesten 70 %, der genannten größten Querbreite der genannten absorbierenden Struktur verteilt sind.

**7.** Absorbierender Artikel nach einem der vorhergehenden Ansprüche, in welchem die genannten Flüssigkeitstransportöffnungen nur in der Längsmitte des genannten Deckblatts verteilt sind.

**8.** Absorbierender Artikel nach einem der vorhergehenden Ansprüche, in welchem die genannten extra-großen Öffnungen nur in der Quermitte auf 80 %, vorzugsweise 65 %, am bevorzugtesten 50 %, der größten Längslänge der genannten zum Träger gerichteten Oberfläche des genannten Deckblatts verteilt sind.

**9.** Absorbierender Artikel nach einem der vorhergehenden Ansprüche, in welchem die genannten großen Öffnungen nur in der Quermitte auf 80 %, vorzugsweise 65 %, am bevorzugtesten 50 %, der größten Längslänge der genannten zum Träger gerichteten Oberfläche des genannten Deckblatts verteilt sind.

**10.** Absorbierender Artikel nach einem der vorhergehenden Ansprüche, in welchem die genannten Flüssigkeitstransportöffnungen nur in der Quermitte des genannten Deckblatts verteilt sind.

**Revendications**

**1.** Article absorbant ayant un axe longitudinal et un axe transversal, ledit article comprenant une feuille de dessus, une feuille de fond et une structure absorbante placée entre ladite feuille de dessus et ladite feuille de fond, ladite feuille de dessus ayant une surface faisant face à l'utilisateur et une surface faisant face au vêtement, et ladite feuille de dessus comprenant une couche pelliculaire de passage présentant des petits, moyens, grands et très grands orifices pour le transport de liquide,

- lesdits petits orifices ayant une surface individuelle comprise dans la plage allant d'une valeur supérieure à 0 $mm^2$ à 0,1 $mm^2$,
- lesdits moyens orifices ayant une surface individuelle comprise dans la plage allant d'une valeur supérieure à 0,1 $mm^2$ à 0,5 $mm^2$,
- lesdits grands orifices ayant une surface individuelle comprise dans la plage allant d'une valeur supérieure à 0,5 $mm^2$ à 1,4 $mm^2$,
- lesdits très grands orifices ayant une surface individuelle comprise dans la plage allant d'une valeur supérieure à 1,4 $mm^2$ à 3 $mm^2$,
- lesdits petits orifices ayant une surface ouverte totale comprise dans la plage allant de 0,1 % à 5 % de la surface totale de ladite couche pelliculaire de passage,
- lesdits moyens orifices ayant une surface ouverte totale comprise dans la plage allant de 1 % à 35 % de la surface totale de ladite couche pelliculaire de passage,
- lesdits grands orifices ayant une surface ouverte totale comprise dans la plage allant de 1 % à 30 % de la surface totale de ladite couche pelliculaire de passage,
- lesdits très grands orifices ayant une surface ouverte totale comprise dans la plage allant de 1 % à 25 % de la surface totale de ladite couche pelliculaire de passage,
- lesdits orifices de transport de liquide ayant une longueur diagonale intérieure la plus grande et une longueur diagonale intérieure la plus petite, le rapport entre lesdites longueurs diagonales intérieures la plus grande et la plus petite étant compris dans la plage allant de 1 à 6,
- lesdits orifices suivant une répartition telle que lesdits très grands orifices ne sont répartis que dans les 90% de la partie centrale longitudinale de la largeur transversale la plus grande de ladite structure absorbante.

**2.** Article absorbant selon la revendication 1, dans lequel au moins quelques-uns desdits orifices de transport de liquide ont des parois intérieures qui pendent au moins de 0,3 mm par rapport à la surface de ladite couche pelliculaire de passage, lesdites parois intérieures pendent dans une direction orientée vers ladite structure absorbante dudit article.

3. Article absorbant selon l'une quelconque des revendications précédentes, dans lequel la surface ouverte totale de tous lesdits orifices de transport de liquide ménagés dans ladite couche pelliculaire de passage est comprise dans la plage allant de 15 % à 40 % de la surface totale de ladite couche pelliculaire de passage.

4. Article absorbant selon l'une quelconque des revendications précédentes, dans lequel ladite feuille de dessus comprend plus d'une couche de passage.

5. Article absorbant selon l'une quelconque des revendications précédentes, dans lequel lesdits très grands orifices ne sont répartis que dans les 80 % de la partie centrale longitudinale, de préférence 70 %, de la largeur transversale la plus grande de ladite structure absorbante.

6. Article absorbant selon l'une quelconque des revendications précédentes, dans lequel lesdits grands orifices ne sont répartis que dans les 90 % de la partie centrale longitudinale, de préférence 80 %, de la manière la plus préférée 70 %, de la largeur transversale la plus grande de ladite structure absorbante.

7. Article absorbant selon l'une quelconque des revendications précédentes, dans lequel lesdits orifices de transport de liquide ne sont répartis que dans la partie centrale longitudinale de ladite feuille de dessus.

8. Article absorbant selon l'une quelconque des revendications précédentes, dans lequel tous lesdits très grands orifices ne sont répartis que dans les 80 % de la partie centrale transversale, de préférence 65 %, de la manière la plus préférée 50 %, de la longueur longitudinale la plus grande de ladite surface faisant face à l'utilisateur de ladite feuille de dessus.

9. Article absorbant selon l'une quelconque des revendications précédentes, dans lequel lesdits grands orifices ne sont répartis que dans les 80 % de la partie centrale transversale, de préférence 65 %, de la manière la plus préférée 50 %, de la longueur longitudinale la plus grande de ladite surface faisant face à l'utilisateur de ladite feuille de dessus.

10. Article absorbant selon l'une quelconque des revendications précédentes, dans lequel lesdits orifices de transport de liquide ne sont répartis que dans la partie centrale transversale de ladite feuille de dessus.